# DEMANDE DE BREVET EUROPEEN

(11) **EP 1 008 329 A1**
(43) Date de publication de la demande: **14.06.2000**
(21) Numéro de dépôt: 99403076.5
(22) Date de dépôt: 08.12.1999
(51) Int. Cl.: A61F 2/06

(54) **Extenseur coronaire ou stent**

(30) Priorité: 08.12.1998 FR 9815490
(71) Demandeur: Stent Tech (S.A.R.L.), 78860 Saint-nom-la-Breteche (FR)
(72) Inventeur: Sabaria, Patrick, c/o Stent Tech (S.A.R.L.), 78860 Saint-Nom-La-Breteche (FR)
(74) Mandataire: Breese, Pierre

(57) **Abrégé**

La présente invention concerne une endoprothèse coronaire de type "stent" présentant la forme générale d'un élément cylindrique allongé expansible cylindrique et constitué d'une pluralité d'éléments (1, 3, 5) de forme générale annulaire et d'une pluralité (2, 4) de bras de liaison (12) qui interconnectent des éléments annulaires (1, 3, 5) adjacents caractérisé en ce que les noeuds de raccordement entre les bras de liaison et les éléments annulaires sont décalés angulairement par rapport aux noeuds de raccordement de la maille suivante.

## Description

La présente invention concerne le domaine des extenseurs vasculaires et coronaires, habituellement désignés sous le nom de "stent".

La configuration générale de ces dispositifs est connue dans l'état de la technique, par exemple par le brevet européen EP556850. Ce brevet décrit un stent intraluminal comprenant une longueur déterminée de fil élastique ayant une configuration sinueuse ou en zigzag et formant une hélice continue ayant une série de spires, et une série d'éléments bouclés joignant les sommets voisins des spires d'hélice voisines. Ce stent est compressible et auto-expansible sensiblement jusqu'à une configuration précomprimée.

Le demandeur à également déposé une demande de brevet français sous le numéro FR97/05516 concernant un stent comportant une alternance de motifs ondulés et de bras en forme de "S" orientés en sens alternés.

Il est apparu que les stents de l'art antérieur présente un recul élastique transversal ("recoil") relativement important. Ce recul est d'autant plus important que des contraintes externes sont appliquées par la paroi de l'endoprothèse.

Ce recul constitue un effet néfaste. S'il n'est pas compensé, il produit un rétrécissement de la lumière vasculaire. Pour éviter un tel rétrécissement, il faut surdilater l'endoprothèse, ce qui produit un impactage néfaste du stent dans l'artère. A titre d'exemple, un recul de 10% habituel pour des endoprothèses de l'état de la technique conduit à une lumière de 2,7 mm pour une dilatation nominale à 3 mm. Ce rétrécissement provoque des problèmes post-implantation.

Le but de la présente invention est de remédier à ces inconvénients en proposant une nouvelle famille d'endoprothèses à recul élastique réduit.

A cet effet, l'invention concerne dans son acception la plus générale un élargisseur périphérique coronaire de type "stent" présentant la forme générale d'un élément cylindrique allongé expansible cylindrique et constitué d'une pluralité d'éléments de forme générale annulaire et d'une pluralité de bras de liaison qui interconnectent des éléments annulaires adjacents caractérisé en ce que les noeuds de raccordement entre les bras de liaison et les éléments annulaires sont décalés angulairement par rapport aux noeuds de raccordement de la maille suivante.

Selon un mode de réalisation préféré, les éléments annulaires sont réalisés par un fil élastique ayant une configuration sinueuse ou en zigzag dont certaines des boucles au moins sont reliées à certaines des boucles de la configuration sinueuse ou en zigzag de l'élément annulaire consécutif par des bras en forme de "S" dont les deux extrémités sont sensiblement alignés sur un axe longitudinal, les axes longitudinaux des bras en "S" de deux séries consécutives étant décalées angulairement.

De préférence, le décalage angulaire entre les axes longitudinaux d'une premier série et les axes longitudinaux d'une série consécutive correspond à un demi-pas de la configuration périodique annulaire.

Selon une variante préférée, les bras (12) en forme de "S" présentent un segment médian incliné par rapport au plan transversal.

Avantageusement, l'épaisseur de la paroi est d'environ 0,09 millimètres.

De préférence, la longueur d'un élément annulaire est comprise entre 1 à 1,2 millimètres.

Avantageusement, la distance entre les sommets de deux éléments annulaires consécutifs est comprise entre 0,7 et 0,8 mm.

Selon un mode de réalisation particulier, la largeur des boucles des segments annulaires est comprise entre 0,12 et 0,14 mm et la largeur des bras de liaison est comprise entre 0,07 et 0,09 mm.

Selon un exemple de réalisation préféré, chaque segment annulaire comporte huit motifs identiques.

Selon une variante avantageuse, l'endoprothèse selon l'invention présente des lumières en forme de "H incliné prolongé latéralement par des branches obliques" présentant un axe principal passant par les branches obliques alignées avec l'axe longitudinal.

Avantageusement, les lumières d'une première série annulaire sont décalées angulairement des lumières de la série annulaire suivante.

L'invention sera mieux comprise à la lecture de la description d'un exemple non limitatif de réalisation qui suit, se référant aux dessins annexés, où :

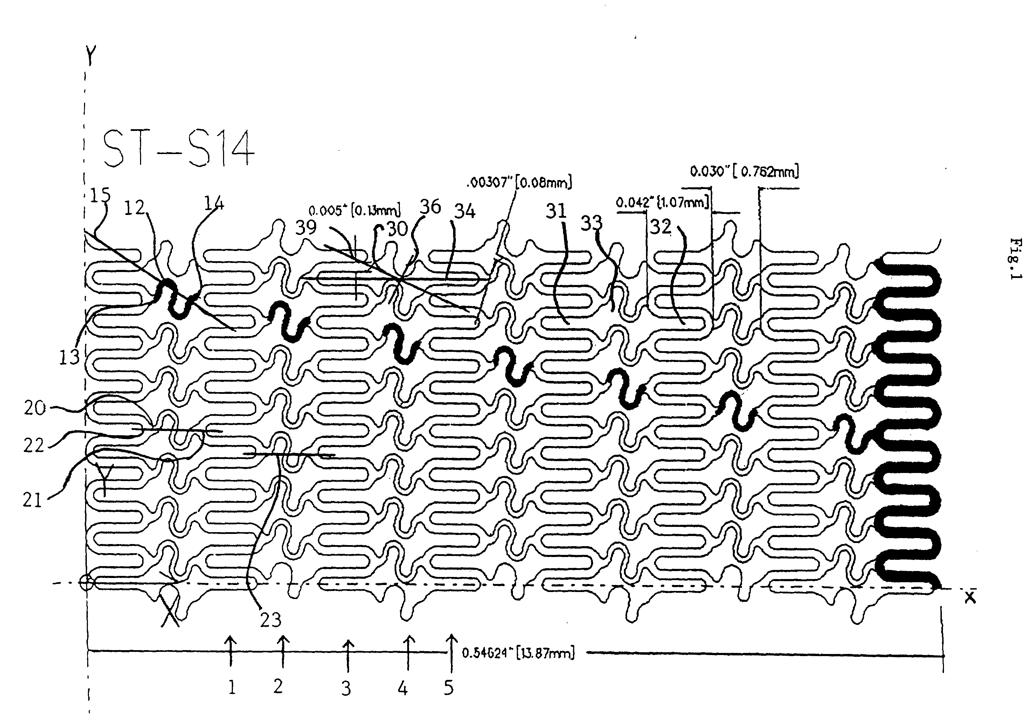

- la figure 1 représente une vue développée du stent.

La figure 1 représente une développée du stent. Il est formé d'une alternance d'éléments de forme extérieure annulaire (1, 3, 5) et d'éléments de grande flexibilité (2, 4).

Les éléments annulaires (1, 3, 5) sont formés par une configuration sinueuse refermée sur elle en forme de couronne. Cette configuration sinueuse est réalisée en acier de type 316 L, découpé au laser et traité par électro-polissage.

Deux éléments annulaires consécutifs (1, 3) sont reliés par une série de bras (12) en forme de "S", dont l'une des extrémités prolonge l'une des boucles (13) du premier élément (1) et dont l'autre extrémité prolonge la boucle (14) de l'élément consécutif faisant sensiblement face à la première boucle (13). En position initiale, l'axe médian (15) du bras (12) forme un angle de l'ordre de 45° avec l'axe longitudinal médian du stent. L'axe médian (15) est défini comme la perpendiculaire à la tangente au point d'inflexion du bras en forme de "S". Le bras en "S" présente deux boucles symétriques s'ouvrant sur 180° environ, contrairement à des boucles en forme de "Ω" dont les boucles se referment sur plus de 180°.

Les axes (22) passant par les deux extrémités (20, 21) des bras en "S" sont alignés avec l'axe longitudinal de l'endoprothèse.

Les axes longitudinaux (22, 23) de deux séries consécutives sont décalées angulairement d'un demi-pas.

A titre d'exemple, la longueur du stent est de 13,87 mm et présente une section avant dilatation de 1,6 mm. Le stent comporte entre 8 et 13 séries de couples de segments annulaires. Les bras (12) se raccordent au sommet des boucles adjacentes.

Les lumières (30) présentent la forme d'un "H incliné (33) prolongé de bras latéraux (31, 32)". L'axe principal (34) et orienté selon l'axe longitudinal de l'endoprothèse. Elles présentent un axe de symétrie médian (35) et un axe de symétrie transversal (36). La forme générale des lumières est isomérique.
L'invention est décrite dans ce qui précède à titre d'exemple non limitatif.

## Revendications

1. Elargisseur périphérique coronaire présentant la forme générale d'un élément cylindrique allongé expansible cylindrique et constitué d'une pluralité d'éléments (1, 3, 5) de forme générale annulaire réalisés par un fil élastique ayant une configuration sinueuse ou en zigzag dont certaines des boucles (13) au moins sont reliées à certaines des boucles de la configuration sinueuse ou en zigzag de l'élément annulaire consécutif par des bras de liaison (12) en forme de "S" qui interconnectent des éléments annulaires (1, 3, 5) adjacents, caractérisé en ce que les axes longitudinaux des bras en "S" de deux séries consécutives sont décalées angulairement et en ce que les bras (12) en forme de "S" présentent un segment médian incliné par rapport au plan transversal.

2. Elargisseur périphérique coronaire selon la revendication 1 caractérisé en ce que le décalage angulaire entre les axes longitudinaux d'une premier série et les axes longitudinaux d'une série consécutive correspond à un demi-pas de la configuration périodique annulaire.

3. Elargisseur périphérique coronaire selon l'une au moins des revendications précédentes caractérisé en ce que l'épaisseur de la paroi est d'environ 0,09 millimètres.

4. Elargisseur périphérique coronaire selon l'une au moins des revendications précédentes caractérisé en ce que la longueur d'un élément annulaire (1, 3, 5) est comprise entre 1 à 1,2 millimètres.

5. Elargisseur périphérique coronaire selon l'une au moins des revendications précédentes caractérisé en ce que la distance entre les sommets de deux éléments annulaires consécutifs est comprise entre 0,7 et 0,8 mm.

6. Elargisseur périphérique coronaire selon l'une au moins des revendications précédentes caractérisé en ce que la largeur des boucles des segments annulaires est comprise entre 0,12 et 0,14 mm.

7. Elargisseur périphérique coronaire selon l'une au moins des revendications précédentes caractérisé en ce que la largeur des bras de liaison est comprise entre 0,07 et 0,09 mm.

8. Elargisseur périphérique coronaire selon l'une au moins des revendications précédentes caractérisé en ce que chaque segment annulaire comporte huit motifs identiques.

9. Elargisseur périphérique coronaire selon l'une au moins des revendications précédentes caractérisé en ce qu'il présente des lumières en forme de "H incliné prolongé latéralement par des branches obliques" présentant un axe principal passant par les branches obliques aligné avec l'axe longitudinal.

10. Elargisseur périphérique coronaire selon la revendication précédente caractérisé en ce que les lumières d'une première série annulaire sont décalées angulairement des lumières de la série annulaire suivante.

11. Elargisseur périphérique coronaire selon l'une au moins des revendications précédentes caractérisé en ce qu'il présente des lumières en forme de "H incliné prolongé latéralement par des branches obliques" présentant un axe principal passant par les branches obliques aligné avec l'axe longitudinal.
